# EUROPEAN PATENT SPECIFICATION

(11) **EP 3 432 388 B1**
(45) Date of publication and mention of the grant of the patent: **19.08.2020**
(21) Application number: 17382465.7
(22) Date of filing: 17.07.2017
(51) Int. Cl.: H01M 4/1393, H01M 4/58, H01M 4/62, H01M 10/052, C07D 233/64

(54) **CATHODE FOR LITHIUM SULFUR BATTERIES**
KATHODE FÜR LITHIUM-SCHWEFEL-BATTERIEN
CATHODE POUR BATTERIES AU LITHIUM/SOUFRE

(43) Date of publication of application: 23.01.2019
(73) Proprietor: Acondicionamiento Tarrasense, 08225 Terrassa Barcelona (ES)
(72) Inventor: VALDIVIELSO PABLO, Ángel M., 08225 TERRASSA (ES); AUCHER, Christophe, 08225 TERRASSA (ES); GUTIÉRREZ-TAUSTE, David, 08225 TERRASSA (ES); AUBOUY, Laurent, 08225 TERRASSA (ES)
(74) Representative: Gallego Jiménez, José Fernando

(56) References cited:
- HANS SCHWARZ ET AL: "The Use of p-Nitrobenzyl Esters in Peptide Synthesis", JOURNAL OF THE AMERICAN CHEMICAL SOCIETY, AMERICAN CHEMICAL SOCIETY, US, vol. 81, no. 21, 5 November 1959 (1959-11-05), pages 5691-5695, XP007906771, ISSN: 0002-7863, DOI: 10.1021/JA01530A041
- WANG XIWEN ET AL: "Nitrogen-doped graphene/sulfur composite as cathode material for high capacity lithium-sulfur batter", JOURNAL OF POWER SOURCES, ELSEVIER SA, CH, vol. 256, 28 January 2014 (2014-01-28), pages 361-368, XP028661744, ISSN: 0378-7753, DOI: 10.1016/J.JPOWSOUR.2014.01.093

## Description

### Technical Field

The present invention relates to a new imidazolium salt suitable for functionalizing carbon materials used as cathode for lithium sulfur batteries.

### Technical Background

Lithium sulfur (Li-S) batteries are subjected to a high number of research topics because they could deliver theoretically 2600 Wh/kg, which is much higher than present state-of the art lithium batteries, whose theoretical and practical energy density is 430 to 570 Wh/kg for LiC₆-LiCoO₂ and 120 to 180 Wh/kg for Li-LiMn₂O₄ systems. Moreover, they are made of relatively inexpensive and non-poisonous materials.

Li-S batteries show specific advantageous features such as: rechargeable, relatively high energy density and specific power, relatively light, operating over a wide temperature range (about -50° C to about 70° C), intrinsic protection mechanism from overcharge, providing safety, relatively inexpensive cathode materials (sulfur), and relatively safe for the environment, in comparison to Li ion batteries.

Li-S batteries generally include a lithium anode, an electrolyte, a porous separator, and a sulfur cathode. In a discharge operation of the battery, the lithium anode is oxidized to form lithium ions, while the sulfur cathode is reduced to form polysulfides.

However, they have several disadvantages which include: a) relatively low practical specific energy (200-300 Wh/kg) against expected values of 450-650 Wh/kg; b) rapid decrease in capacity during cycling (0.1-0.4% per cycle); and c) high self-discharge rates (8-15% per month), as disclosed in Kolosnitsyn et al., Lithium-Sulfur Batteries: Problems and Solutions, Russian J. Electrochem., 2008, 44(5), 506-509.

These disadvantages are caused by the low conductivity of sulfur and its final discharge products, expansion of the cathode upon lithiation and the solubility of sulfur and of lithium polysulfides in battery electrolytes. The expansion of the cathode occurs because the molar volumes of lithium sulfide and polysulfides are about 80% greater than that of sulfur. Partial dissolution of polysulfides leads to anode corrosion and to the formation of shorter polysulfide compounds. Short polysulfides, in turn, diffuse and migrate to the cathode where they re-oxidize or react with the solid sulfur at the cathode to form longer polysulfides, initiating a shuttle mechanism, which leads to low energy-conversion efficiency. The migration of polysulfides through the separator and their reaction on a surface of the anode, causes further performance and capacity degradation, as disclosed in Bresser et al., Recent progress and remaining challenges in sulfur-based lithium secondary batteries - a review, Chem. Commun., 2013, 49(90), 10545-10562.

Various attempts have been made to address the poor cycling life and rate performance with conventional Li-S batteries.

One approach is to encapsulate sulfur with conducting materials such as mesoporous carbon as disclosed in Ji et al., A highly ordered nanostructured carbon-sulphur cathode for lithium-sulphur batteries, Nat. Met., 2009, 8, 500-506.

Another approach disclosed in Xin et al., Smaller Sulfur Molecules Promise Better Lithium-Sulfur Batteries, J. Am. Chem. Soc., 2012, 134, 18510-1818513, refers to the synthesis of small sulfur molecules in the confined space of a microporous carbon matrix avoids the unfavorable formatin of long-chain polysulfides, and hence avoiding the dissolution problem.

Another technique includes modifying the cathode to avoid the polysulfide migration through the porous separator to the surface anode and stabilise the system. There are several technical solutions proposed in the art. For example in Liu et al., Nanostructured Metal Oxides and Sulfides for Lithium-Sulfur Batteries, Adv. Mater. 2017, 1601759 it is disclosed the nanoencapsulation of sulfur, for example using Ti₄O₇, whereas in Qian et al., Zn-MOF derived micro/meso porous carbon nanorod for high performance lithium-sulfur battery, RSC Adv., 2016, 6 (97), 94629, it is disclosed the impregnation into a porous material, like zinc metal-organic framework (MOF).

Taking into account that the physical entrapment of polysulfide compounds alone is not sufficient to prevent the dissolution of those compounds into the electrolyte, another approach follows the modification of the sulfur cathode to improve the chemical binding with the polysulfide species. For example, in Ji et al., Graphene Oxide as a Sulfur Immobilizer in High Performance Lithium/Sulfur Cells, J. Am. Chem. Soc., 2011, 133, 18522-18525, it is disclosed the use of graphene oxide, having oxygen rich groups, to immobilize sulfur and polysulfide species in Li-S batteries.

The improvement of electrochemical properties of a Li-S battery is disclosed in Wang et al., Nitrogen-doped graphene/sulfur composite as cathode material for high capacity lithium-sulfur batteries, J. Power Sources, 2014, 256, 361-368, wherein nitrogen-doped graphene sheets were used to immobilize sulfur via *in situ* sulfur deposition.

The production of nitrogen-doped carbon materials to improve enhanced chemical adsorption ability is disclosed also in Ding et al., Facile Solid-State Growth of 3D Well-Interconnected Nitrogen-Rich Carbon Nanotube-Graphene Hybrid Architectures for Lithium-Sulfur Batteries, Adv. Funct. Mater., 2016, 26, 1112-1119.

Another approach to improve Li-S batteries is disclosed in Xie et al., Ferroelectric-Enhanced Polysulfide Trapping for Lithium-Sulfur Battery Improvement, Adv. Mat., 2016, 201604724, wherein the addition of a ferroelectric material, such as BaTiO₃ (BTO), in the form of nanoparticles into the battery cathode anchors the polysulfides, preventing them from dissolving and causing the loss of active materials at the cathode.

New cathode materials including a metal oxide or oxynitride with a porous structure are disclosed in International patent application WO-A-2017/091271, and a sulfur-carbon composite comprising a porous highly graphitic carbon material and sulfur for preparing a cathode of a Li-S battery is disclosed in International patent application WO-A-2017/079976.

In International patent application WO-A-2017/052246 it is disclosed a cathode, which comprises metal nanoparticles increasing the reactivity of sulfur and the electric capacity of the cathode. Further, battery reaction products such as lithium sulfide are easily decomposed by a catalytic reaction such that lifespan characteristics can be improved.

Another approach to improve Li-S batteries is disclosed in International patent application WO-A-2016/094301, wherein a multilayered cathode is used, which mitigates the capacity fading and low power density problems that exists in sulfur cathodes.

Thus, in the prior art there are more or less complex approaches to improve the performance of Li-S batteries.

However, there is a need of providing new cathodes for Li-S batteries, prepared from easily available compounds by processes being easily scalable and without handling problems.

### Object of the invention

The object of the present invention is a compound of general formula (I).

Also part of the invention is a process for preparing compound of general formula (I).

Also part of the invention is a process for preparing a functionalized carbon material of general formula (VI) from compound of general formula (I) and a carbon material.

Also part of the invention is a functionalized carbon material of general formula (VI) obtainable by such process.

Also part of the invention is a composite comprising the functionalized material of general formula (VI) and sulfur.

Also part of the invention is an aqueous ink comprising that composite.

Also part of the invention is a cathode comprising that functionalized carbon material.

Also part of the invention is the use of that functionalized carbon material for preparing a cathode.

Also part of the invention is a lithium sulfur battery comprising that functionalized carbon material.

Also part of the invention is a lithium sulfur battery comprising that cathode.

Also part of the invention is the use of that functionalized carbon material for preparing a lithium sulfur battery.

### Brief description of drawings:

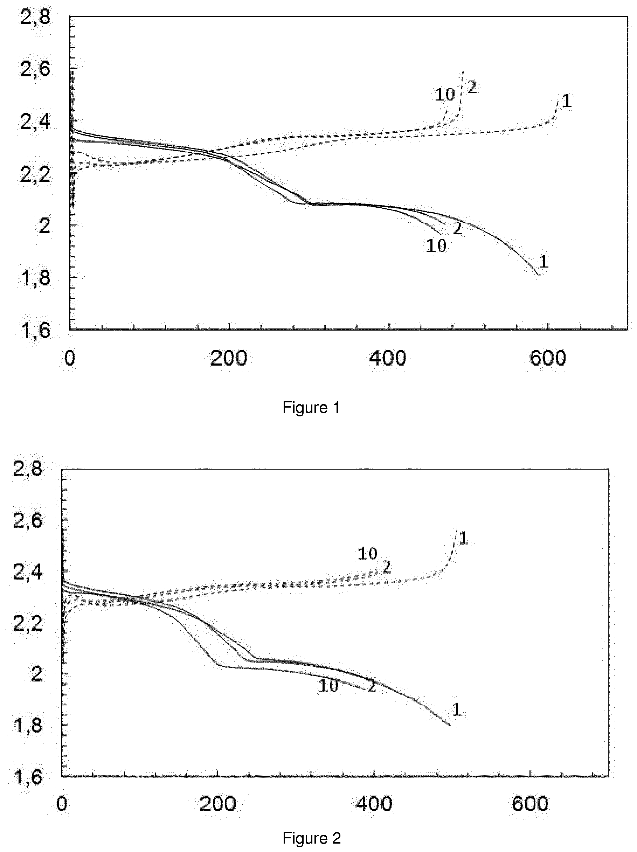

Figure 1 represents the discharge and charge voltage curves for selected cycles (1, 2 and 10) of a battery comprising a cathode comprising functionalized carbon material and functionalized conductive additive according to the invention, prepared as disclosed in Example 9. In ordinates, voltage vs Li⁺/Li is represented, and in abscissas the specific capacity in mA.h.g⁻¹ is represented.
Figure 2 represents the discharge and charge voltage curves for selected cycles (1, 2 and 10) of a battery comprising a cathode comprising comparative non-functionalized carbon material and non-functionalized conductive additive, prepared as disclosed in Example 11. In ordinates, voltage vs Li⁺/Li is represented, and in abscissas the specific capacity in mA.h.g⁻¹ is represented.

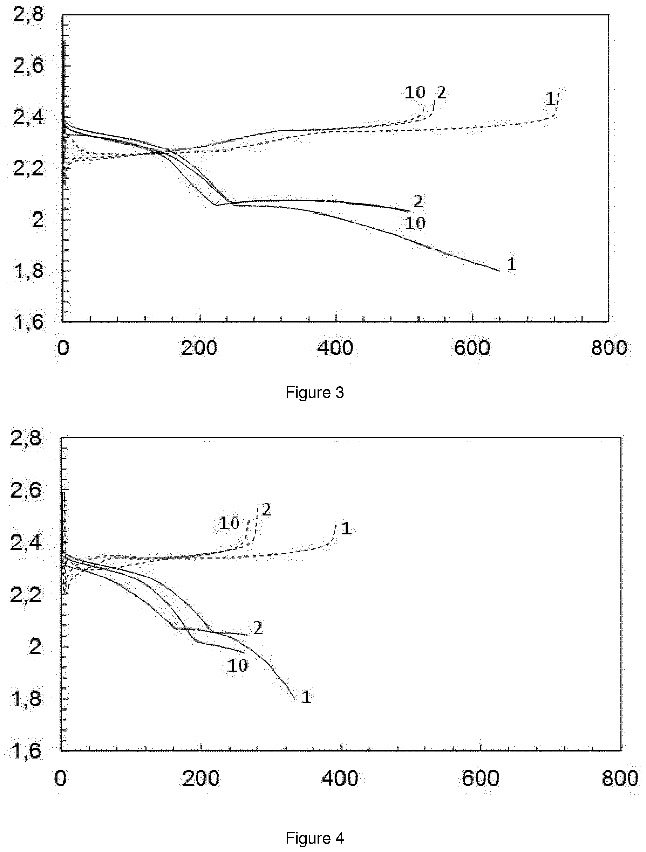

Figure 3 represents the discharge and charge voltage curves for selected cycles (1, 2 and 10) of a battery comprising a cathode comprising functionalized carbon material and functionalized conductive additive according to the invention, prepared as disclosed in Example 10. In ordinates, voltage vs Li⁺/Li is represented, and in abscissas the specific capacity in mA.h.g⁻¹ is represented.
Figure 4 represents the discharge and charge voltage curves for selected cycles (1, 2 and 10) of a battery comprising a cathode comprising comparative non-functionalized carbon material and non-functionalized conductive additive, prepared as disclosed in Example 12. In ordinates, voltage vs Li⁺/Li is represented, and in abscissas the specific capacity in mA.h.g⁻¹ is represented.

### Detailed description of the invention

The object of the present invention is compound of general formula (I): 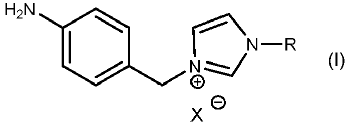 wherein:
- R is linear or branched C₁-C₂₀ alkyl, or alicyclic C₃-C₁₀, and
- X⁻ is bromide (Br⁻), chloride (Cl⁻), bis[(trifluoromethyl)sulfonyl]imide ((CF₃SO₂)₂N⁻, TFSI), or bis(fluorosulfonyl)imide ((FSO₂)N⁻, FSI).

The authors of the present invention have developed compound of general formula (I), which surprisingly is useful for modifying the surface of carbon materials such as graphite powder, graphite fibers, carbon fibers, carbon cloth, vitreous carbon products, activated carbon products, or carbon nanotubes, to produce functionalized compounds, which are suitable for preparing cathodes for lithium-sulfur batteries by an easily scalable process, avoiding the use chemical reactions with expensive and sensitive reagents and electrochemical reactions. The advantages of the compound of general formula (I) is that commercial and inexpensive materials are used for preparing suitable materials for manufacturing cathodes. Moreover, the attachment or grafting of compound of general formula (I) on a carbon material is carried out in a single step, using inexpensive chemical reagents and solvent, and avoiding any electrochemical process.

In the present description as well as in the claims, the singular forms "a" and "an" include also the plural reference unless the context clearly indicates otherwise.

In this description, the percentages (%) are expressed in weight/weight unless otherwise indicated, and in the compositions, the sum of the percentages of the different components is adjusted to add up to 100%.

### Compound of general formula (I)

Compound of general formula (I) forms part of the present invention.

In a preferred embodiment, R is selected from linear or branched C₁-C₂₀ alkyl, more preferably from linear or branched C₁-C₁₀ alkyl, more preferably from linear or branched C₁-C₅ alkyl, more preferably from linear C₁-C₄ alkyl, and yet more preferably is methyl. In a preferred embodiment, X⁻ is bromide. In another preferred embodiment, X⁻ is chloride. In another preferred embodiment X⁻ is (CF₃SO₂)₂N⁻. In another preferred embodiment X⁻ is (FSO₂)N⁻.

In a preferred embodiment, R is selected from linear or branched C₁-C₂₀ alkyl, and X⁻ is bromide (Br⁻), chloride (Cl⁻), (CF₃SO₂)₂N⁻, or (FSO₂)N⁻, more preferably bromide.

In a more preferred embodiment, R is selected from linear or branched C₁₋C₁₀ alkyl, and X⁻ is bromide (Br⁻), chloride (Cl⁻), (CF₃SO₂)₂N⁻, or (FSO₂)N⁻, more preferably bromide.

In a yet more preferred embodiment, R is selected from linear or branched C₁-C₅ alkyl, and X⁻ is bromide (Br⁻), chloride (Cl⁻), (CF₃SO₂)₂N⁻, or (FSO₂)N⁻, more preferably bromide.

In a yet more preferred embodiment, R is selected from linear C₁-C₄ alkyl, and X⁻ is bromide (Br⁻), chloride (Cl⁻), (CF₃SO₂)₂N⁻, or (FSO₂)N⁻, more preferably bromide.

In a yet more preferred embodiment, R is methyl, and X⁻ is bromide (Br⁻), chloride (Cl⁻), (CF₃SO₂)₂N⁻, or (FSO₂)N⁻, more preferably bromide.

In another preferred embodiment, R is alicyclic C₃-C₁₀, preferably C₃-C₆, and X⁻ is bromide (Br⁻), chloride (Cl⁻), (CF₃SO₂)₂N⁻, or (FSO₂)N⁻, more preferably bromide.

Compound of general formula (I) can be prepared using standard methods available to the organic chemist. For example, it can be prepared according to the synthetic route shown in Scheme 1:

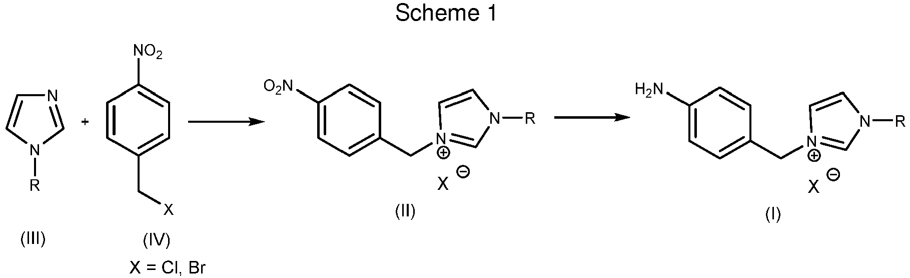

The process for preparing compound of general formula (I) comprises the reaction of compound of general formula (III) 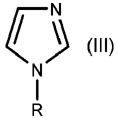 with compound of general formula (IV) 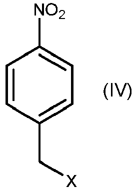 wherein:
- R is linear or branched C₁-C₂₀ alkyl, or alicyclic C₃-C₁₀, and
- X is Br or Cl,
to produce compound of general formula (II) 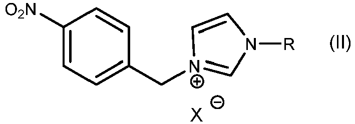 , which is subsequently reduced to produce compound of general formula (I)

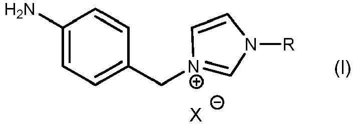

In the first step, alkyl imidazole (III) is reacted with nitroderivative (IV) to yield the compound of general formula (II), which is reduced to compound of general formula (I).

Alkylimidazoles of general formula (III) are readily available compounds, for example, by alkylation of imidazoles as disclosed in Lopez-Pestaña et al., Macroporous and Mesoporous Mat., 2004, 67, 87-94. Some of them can be purchased because they are commercially available, for example, 1-methylimidazole, 1-ethylimidazole, 1-propylimidazole, 1-cyclopropylimidazole, 1-butylimidazole, 1-pentylimidazole, 1-hexylimidazole, 1-cyclohexylimidazole, 1-octylimidazole, 1-dodecylimidazole, 1-tetradecyliizadole, or 1-stearylimidazole.

Nitroderivatives of general formula (IV) can be prepared following standard methods for organic synthesis, or even purchased because they are commercially available, for example, 4-nitrobenzyl bromide.

In the first step, i.e. the coupling step, it is normally used a solvent selected from the group consisting of ethyl acetate, dichloromethane, tetrahydrofuran or acetonitrile. The reaction is usually carried out at a temperature comprised from 20º C to 100º C, preferably from 40º C to 80º C, and more preferably from 60º C to 70º C. in the case of using ethyl acetate, the reaction is carried out preferably at a temperature of 65º C. The reaction is preferably carried out under an inert atmosphere, for example, under nitrogen or argon. The condensed product of general formula (II) is usually isolated from the reaction mixture by precipitation and filtration. The product, normally a solid, is washed with cold solvent and dried under vacuum at a temperature comprised from 20º C to 70º C, preferably from 30º C to 60º C, more preferably from 45º C to 55 º C, and more preferably at a temperature of 50º C. If necessary, it can be further purified by recrystallization from a suitable solvent.

In the second step, i.e. the reduction step, compound of general formula (II) is reduced to the amino derivative compound of general formula (I) by using well known methods to transform a nitro group into an amino group. For example, by using hydrazine in the presence of iron oxides catalyst, as disclosed in Lauwiner et al., J. Appl. Catal. A Gen., 1998, 172, 141-148. Catalyst is removed, once the reaction has been completed, for example, by filtration, and compound of general formula (I) is usually isolated by precipitation and filtration or by removing the solvent by distillation. If necessary, it can be further purified by recrystallization from a suitable solvent.

The process for preparing compound of general formula (I), wherein X⁻ is bis[(trifluoromethyl)sulfonyl]imide ((CF₃SO₂)₂N⁻, TFSI), or bis(fluorosulfonyl)imide ((FSO₂)N⁻, FSI), comprises a further step wherein the halogenide anion, bromide or chloride, is substituted by TFSI or FSI in a methatesis reaction in the presence of an alkaline salt of TFSI or FSI.

The methatesis reaction can be carried out using standard procedures well known to the skilled in the art. For example, to a solution of bromide of general formula (I) in water can be added a solution of LiTFSI or LFSI in water. Compound of general formula (I), wherein X⁻ is TFSI or FSI can be extracted with an organic solvent, such as dichloromethane, and isolated by known methods.

### Functionalized carbon material of general formula (VI)

A process for preparing a functionalized carbon material of formula general (VI) forms also part of the invention:

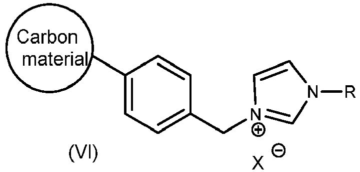

The process comprises the reaction of compound of general formula (I) 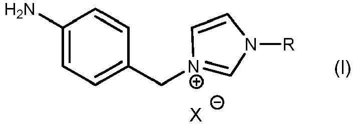 of the invention with a carbon material.

Carbon material suitable for reacting with the compound of formula general (I) is selected from the group consisting of carbon black, carbon nanotubes, multi-walled carbon nanotubes, activated carbon, graphite powder, graphite fiber, graphene, carbon fiber, carbon cloth, and vitreous carbon. Preferably the carbon material is selected from carbon black and multi-walled carbon nanotubes.

The process comprises the generation of a diazonium salt from the compound of general formula (I) and the reaction of the generated diazonium salt with the carbon material. In a preferred embodiment, this diazonium salt is produced from compound of general formula (I), by reaction with an organic or inorganic nitrite and an inorganic acid, for example, hydrochloric acid. The diazonium compound reacts with the carbon material (V) producing a functionalized carbon material (VI), which contains the imidazolium group attached by means of a covalent bond, as shown in Scheme 2:

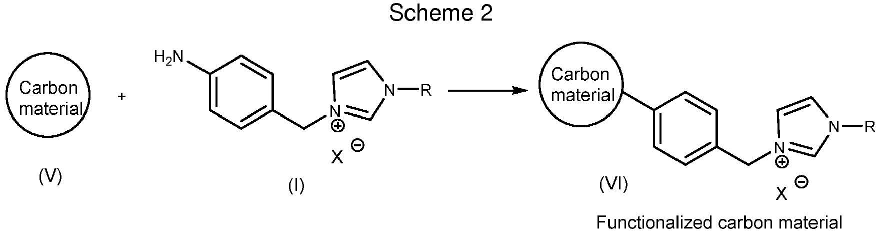

This process can be carried out under a variety of reaction conditions and in any type of reaction medium, including both protic and aprotic solvent systems or slurries as disclosed, for example, in International patent application WO-A-96/18690. Preferably, diazonium salt is formed *in situ.* If desired, the carbon product can be isolated and dried by means known in the art. Furthermore, the resultant carbon product can be treated to remove impurities by known techniques. Preferred embodiments of that process are discussed below and are shown in the examples.

The resulting functionalized carbon material of general formula (VI) is useful in applications known for untreated carbon materials, in particular the preparation of cathodes for Li-S batteries, showing advantageous properties not associated with untreated carbon materials.

The attachment process can be carried out under a wide variety of conditions and in general are not limited by any particular condition. The reaction is carried out usually under acidic conditions, and the temperature may preferably range from 0° C to 100° C. Diazonium salts, as known in the art, may be formed for example by the reaction of primary amines with aqueous solutions of nitrous acid. A general discussion of diazonium salts and methods for their preparation is found, for example, in March's, Advanced Organic Chemistry; Reactions. Mechanisms, and Structure, 5th Ed., 2001, Wiley.

In that process, the diazonium salt may be prepared prior to reaction with the carbon material or, more preferably, generated *in situ* using techniques known in the art. In a particularly preferred process, both the nitrous acid and the diazonium salt are generated *in situ.* A diazonium salt, as is known in the art, may be generated by reacting a primary amine, a nitrite and an acid. The nitrite may be any metal nitrite, preferably lithium nitrite, sodium nitrite, potassium nitrite, or zinc nitrite, or any organic nitrite such as for example isoamylnitrite or ethylnitrite. The acid may be any acid, inorganic or organic, which is effective in the generation of the diazonium salt. Preferred acids include nitric acid, hydrochloric acid, and sulfuric acid.

The process can be carried out in any reaction medium, which allows the reaction between the diazonium salt and the carbon material to proceed. Preferably, the reaction medium is a solvent-based system. The solvent may be a protic solvent, an aprotic solvent, or a mixture of solvents. Protic solvents are solvents, like water or methanol, containing a hydrogen atom attached to an oxygen atom or nitrogen atom and thus are sufficiently acidic to form hydrogen bonds. Aprotic solvents are solvents which do not contain acidic hydrogen. Aprotic solvents include, for example, solvents such as hexanes, tetrahydrofuran (THF), acetonitrile, and benzonitrile.

The process is preferably carried out in a protic reaction medium, that is in a protic solvent alone or a mixture of solvents which contains at least one protic solvent. Preferred protic media include, but are not limited to water, aqueous media containing water and other solvents, alcohols, and any media containing an alcohol, or mixtures of such media.

In general, the process of the invention produces inorganic by-products, such as salts. These by-products may be undesirable and problematic in some cases. Several possible ways to produce a carbon product according to a process of the invention without unwanted inorganic by-products or salts are as follows. First, the diazonium salt can be purified before use by removing the unwanted inorganic by-products using means known in the art. Second, the diazonium salt can be generated with the use of an organic nitrite as the diazotization agent yielding the corresponding alcohol rather than an inorganic salt. Third, the inorganic by-products may be removed by washing with a suitable solvent. Other ways may be known to those of skill in the art.

In addition to the inorganic by-products, a process of the invention may also produce organic by-products. They can be removed, for example, by extraction with organic solvents. Other ways may be known to those of skill in the art.

The percentage of functionalization of the carbon material depends usually on the carbon material, the ratio between compound of general formula (I) and the carbon material, expressed in wt%, and from the concentration of the compound of general formula (I) in the reaction mixture.

Preferably the functionalized carbon material of general formula (VI) is selected from carbon black and multi-walled carbon nanotubes, wherein R is selected from linear or branched C₁-C₂₀ alkyl, more preferably from linear or branched C₁-C₁₀ alkyl, yet more preferably from linear or branched C₁-C₅ alkyl, yet more preferably from linear C₁-C₄ alkyl, and yet more preferably is methyl. In a preferred embodiment, X⁻ is bromide. In another preferred embodiment, X⁻ is chloride. In another preferred embodiment X⁻ is (CF₃SO₂)₂N⁻. In another preferred embodiment X⁻ is (FSO₂)N⁻. In a preferred embodiment, R is selected from linear or branched C₁-C₂₀ alkyl, and X⁻ is bromide (Br⁻), chloride (Cl⁻), (CF₃SO₂)₂N⁻, or (FSO₂)N⁻, more preferably bromide. In a more preferred embodiment, R is selected from linear or branched C₁₋C₁₀ alkyl, and X⁻ is bromide (Br⁻), chloride (Cl⁻), (CF₃SO₂)₂N⁻, or (FSO₂)N⁻, more preferably bromide. Ina yet more preferred embodiment, R is selected from linear or branched C₁-C₅ alkyl, and X⁻ is bromide (Br⁻), chloride (Cl⁻), (CF₃SO₂)₂N⁻, or (FSO₂)N⁻, more preferably bromide. In a yet more preferred embodiment, R is selected from linear C₁-C₄ alkyl, and X⁻ is bromide (Br⁻), chloride (Cl⁻), (CF₃SO₂)₂N⁻, or (FSO₂)N⁻, more preferably bromide. In a yet more preferred embodiment, R is methyl, and X⁻ is bromide (Br⁻), chloride (Cl⁻), (CF₃SO₂)₂N⁻, or (FSO₂)N⁻, more preferably bromide.

In a preferred embodiment the functionalized carbon material of general formula (VI) is carbon black, R is methyl, and X⁻ is bromide (Br⁻), chloride (Cl⁻), (CF₃SO₂)₂N⁻, or (FSO₂)N⁻, more preferably bromide.

In another embodiment the functionalized carbon material of general formula (VI) is multi-walled carbon nanotubes, R is methyl, and X⁻ is bromide (Br⁻), chloride (Cl⁻), (CF₃SO₂)₂N⁻, or (FSO₂)N⁻, more preferably bromide.

Usually the ratio between compound of general formula (I) and carbon material, expressed in wt%, is comprised between 3% and 60%, preferably between 4% and 20%, more preferably between 5% and 7%, and yet more preferably 6%.

Usually the concentration of compound of general formula (I) in the reaction mixture is comprised between 2 mM and 25 mM, preferably between 4 mM and 20 mM, more preferably between 6 mM and 10 mM, and yet more preferably between 7 mM and 8 mM.

In a preferred embodiment, the ratio between compound of general formula (I) and carbon material, expressed in wt%, is comprised between 5% and 7%, and the concentration of compound of general formula (I) in the reaction mixture is comprised between 7 mM and 8 mM.
in a more preferred embodiment, the ratio between compound of general formula (I) and carbon material, expressed in wt%, is about 6%, and the concentration of compound of general formula (I) in the reaction mixture is about 7.5 mM.

Carbon black is commercially available, for example, from Akzo Nobel (USA), and Merck (Germany). Carbon nanotubes, including multi-walled, are available from, for example, Nanocyl (Belgium) and Tokyo Chemical Industry (Japan). Activated carbon and graphite powder are commercially available from, for example, Merck (Germany). Graphite fibers are commercially available from, for example, Nippon Graphite Fibers (Japan). Carbon fibers are commercially available from, for example, Fibre Glast (USA). Carbon cloth is commercially available from, for example, Easy Composites (United Kingdom). Vitreous carbon is commercially available from, for example, Structure Prove (USA).

It forms also part of the invention the functionalized carbon material of general formula (VI) obtainable according to the above exposed process.

### Composite comprising functionalized material of general formula (VI) and sulfur

The composite comprising the functionalized material of general formula (VI) of the invention, and sulfur forms also part of the invention.

Preferably, the functionalized material of general formula (VI) is functionalized carbon material selected from the group consisting of carbon black, carbon nanotubes, multi-walled carbon nanotubes, activated carbon, graphite powder, graphite fiber, graphene, carbon fiber, carbon cloth, and vitreous carbon, more preferably selected from carbon black and multi-walled carbon nanotubes, wherein R is selected from linear or branched C₁-C₂₀ alkyl, more preferably from linear or branched C₁-C₁₀ alkyl, yet more preferably from linear or branched C₁-C₅ alkyl, yet more preferably from linear C₁-C₄ alkyl, and yet more preferably is methyl. In a preferred embodiment, X⁻ is bromide. In another preferred embodiment, X⁻ is chloride. In another preferred embodiment X⁻ is (CF₃SO₂)₂N⁻. In another preferred embodiment X⁻ is (FSO₂)N⁻. In a preferred embodiment, R is selected from linear or branched C₁-C₂₀ alkyl, and X⁻ is bromide (Br⁻), chloride (Cl⁻), (CF₃SO₂)₂N⁻, or (FSO₂)N⁻, more preferably bromide. In a more preferred embodiment, R is selected from linear or branched C₁₋C₁₀ alkyl, and X⁻ is bromide (Br⁻), chloride (Cl⁻), (CF₃SO₂)₂N⁻, or (FSO₂)N⁻, more preferably bromide. Ina yet more preferred embodiment, R is selected from linear or branched C₁-C₅ alkyl, and X⁻ is bromide (Br⁻), chloride (Cl⁻), (CF₃SO₂)₂N⁻, or (FSO₂)N⁻, more preferably bromide. In a yet more preferred embodiment, R is selected from linear C₁-C₄ alkyl, and X⁻ is bromide (Br⁻), chloride (Cl⁻), (CF₃SO₂)₂N⁻, or (FSO₂)N⁻, more preferably bromide. In a yet more preferred embodiment, R is methyl, and X⁻ is bromide (Br⁻), chloride (Cl⁻), (CF₃SO₂)₂N⁻, or (FSO₂)N⁻, more preferably bromide.

In a preferred embodiment the functionalized carbon material of general formula (VI) is carbon black, R is methyl, and X⁻ is bromide (Br⁻), chloride (Cl⁻), (CF₃SO₂)₂N⁻, or (FSO₂)N⁻, more preferably bromide.

In another embodiment the functionalized carbon material of general formula (VI) is multi-walled carbon nanotubes, R is methyl, and X⁻ is bromide (Br⁻), chloride (Cl⁻), (CF₃SO₂)₂N⁻, or (FSO₂)N⁻, more preferably bromide.

The composite may be prepared by a process, which comprises the following steps:
1) homogenizing functionalized carbon material of general formula (VI) and sulfur in powdery form, preferably by mortar, and more preferably using hexane for preparing an slurry until hexane evaporation, and
2) heating the homogenized mixture obtained in step 1) at a temperature comprised between 120º C and 200º C, preferably between 140º C to 180º C, and more preferably between 150º C and 160º C.

The heating step facilitates the melt infiltration of sulfur into the porous surface of the carbon material.

Usually, the composite shows a particle size distribution of D10 about 4-5 µm, D50 about 10-12 µm and D90 about 55-65 µm, being the particle size distribution determined in water following standard procedures, such as Malvern light scattering.

The composite comprises from 60 wt% to 90 wt%, preferably from 65 wt% to 85 wt%, more preferably from 70 wt% to 82 wt%, more preferably from 75 wt% to 80 wt%, and yet more preferably 79 wt% of sulfur.

### Aqueous ink

An aqueous ink comprising the composite comprising the functionalized carbon material of general formula (VI) of the invention, and sulfur forms also part of the present invention.

The aqueous ink comprises:
a) the composite comprises functionalized carbon material of general formula (VI) of the invention and sulfur,
b) a conductive additive,
c) a stabilizing agent,
d) a binder, and
e) water as a solvent

A conductive additive is a compound selected from the group consisting of carbon black, carbon nanotubes, multi-walled carbon nanotubes, activated carbon, graphite powder, graphite fiber, graphene, carbon fiber, carbon cloth, and vitreous carbon. Preferably the conductive additive is selected from carbon black and multi-walled carbon nanotubes.

In a preferred embodiment, the conductive additive is the functionalized carbon material of general formula (VI), as disclosed above.

Preferably, the functionalized material of general formula (VI) is functionalized carbon material selected from the group consisting of carbon black, carbon nanotubes, multi-walled carbon nanotubes, activated carbon, graphite powder, graphite fiber, graphene, carbon fiber, carbon cloth, and vitreous carbon, more preferably selected from carbon black and multi-walled carbon nanotubes, wherein R is selected from linear or branched C₁-C₂₀ alkyl, more preferably from linear or branched C₁₋C₁₀ alkyl, yet more preferably from linear or branched C₁-C₅ alkyl, yet more preferably from linear C₁-C₄ alkyl, and yet more preferably is methyl. In a preferred embodiment, X⁻ is bromide. In another preferred embodiment, X⁻ is chloride. In another preferred embodiment X⁻ is (CF₃SO₂)₂N⁻. In another preferred embodiment X⁻ is (FSO₂)N⁻. In a preferred embodiment, R is selected from linear or branched C₁-C₂₀ alkyl, and X⁻ is bromide (Br⁻), chloride (Cl⁻), (CF₃SO₂)₂N⁻, or (FSO₂)N⁻, more preferably bromide. In a more preferred embodiment, R is selected from linear or branched C₁-C₁₀ alkyl, and X⁻ is bromide (Br⁻), chloride (Cl⁻), (CF₃SO₂)₂N⁻, or (FSO₂)N⁻, more preferably bromide. Ina yet more preferred embodiment, R is selected from linear or branched C₁-C₅ alkyl, and X⁻ is bromide (Br⁻), chloride (Cl⁻), (CF₃SO₂)₂N⁻, or (FSO₂)N⁻, more preferably bromide. In a yet more preferred embodiment, R is selected from linear C₁-C₄ alkyl, and X⁻ is bromide (Br⁻), chloride (Cl⁻), (CF₃SO₂)₂N⁻, or (FSO₂)N⁻, more preferably bromide. In a yet more preferred embodiment, R is methyl, and X⁻ is bromide (Br⁻), chloride (Cl⁻), (CF₃SO₂)₂N⁻, or (FSO₂)N⁻, more preferably bromide.

In a preferred embodiment the functionalized carbon material of general formula (VI) is carbon black, R is methyl, and X⁻ is bromide (Br⁻), chloride (Cl⁻), (CF₃SO₂)₂N⁻, or (FSO₂)N⁻, more preferably bromide.

In another embodiment the functionalized carbon material of general formula (VI) is multi-walled carbon nanotubes, R is methyl, and X⁻ is bromide (Br⁻), chloride (Cl⁻), (CF₃SO₂)₂N⁻, or (FSO₂)N⁻, more preferably bromide.

The stabilizing agent is selected form the group consisting of sodium carboxymethyl cellulose, xanthan gum, hydroxypropylmethyl cellulose, carbomer, polyvinylpyrrolidone, and mixtures thereof. Preferably the stabilizing agent is sodium carboxymethyl cellulose. The stabilizing agent confers rheological properties to the ink in order to stabilize the dispersion of the composite and the conductive additive.

The binder is selected from: styrene butadiene rubber copolymer, poly(vinylidene fluoride) (PVDF-HFP), polyvinylpyrrolidone (PVP), mix of PVP with polyethylene imine (PEI), polyaniline (PANI) and lithium polyacrylate (LiPAA). In a preferred embodiment the binder is a water based styrene-butadiene rubber (SBR) copolymer. In a more preferred embodiment, the SBR binder has a solid content comprised between 40 wt% and 60 wt%, preferably between 45 wt% and 55 wt%, and more preferably between 48 wt% and 52 wt%. In a preferred embodiment the binder shows a glass transition temperature comprised from -10º C to -25º C, preferably from - 15º C to 21º C, more preferably from -17º C to -19º C, and yet more preferably -18º C. In a preferred embodiment the viscosity measured according to NDJ-55 at 25º C is comprised between 50 and 500 mPa.s, more preferably between 100 and 350 mPa.s, Usually the content of styrene is comprised between 23 wt% to 25 wt%, the content of butadiene is comprised between 70 wt% and 72 wt%, and the content of carboxylic groups is about 5 wt%.

SBR binders are commercially available for example through the companies MTI (USA) or Targray (USA).

The solid content of the aqueous ink comprises from 80 wt% to 90 wt%, preferably from 82 wt% to 88 wt%, more preferably from 84 wt% to 86 wt%, and yet more preferably 85 wt% of the composite of functionalized carbon material of general formula (VI) and sulfur; from 2 wt% to 10 wt%, preferably from 3 wt% to 8 wt%, more preferably from 4 wt% to 6 wt%, and yet more preferably 5 wt% of the conductive additive; from 2 wt% to 10 wt%, preferably from 3 wt% to 8 wt%, more preferably from 4 wt% to 6 wt%, and yet more preferably 5 wt% of the stabilizing agent, preferably sodium carboxymethyl cellulose; and from 2 wt% to 10 wt%, preferably from 3 wt% to 8 wt%, more preferably from 4 wt% to 6 wt%, and yet more preferably 5 wt% of binder, expressed as solids, wherein the sum of percentages of the solid components of the aqueous ink amounts to 100 wt%. In a preferred embodiment the solid content of the aqueous ink comprises:
a) 85 wt% of the composite comprising the functionalized carbon material of general formula (VI), wherein R is linear or branched C₁-C₂₀ alkyl, or alicyclic C₃-C₁₀, and X⁻ is bromide (Br⁻), (Cl⁻), bis[(trifluoromethyl)sulfonyl]imide ((CF₃SO₂)₂N⁻, TFSI), or bis(fluorosulfonyl)imide ((FSO₂)N⁻, FSI), and sulfur, preferably functionalized carbon black of general formula (VI), more preferably functionalized carbon black of general formula (VI), wherein R is methyl and X⁻ is bromide,
b) 5 wt% of conductive additive, preferably functionalized multi-walled carbon nanotubes of general formula (VI), wherein R is linear or branched C₁-C₂₀ alkyl, or alicyclic C₃-C₁₀, and X⁻ is bromide (Br), chloride (Cl⁻), bis[(trifluoromethyl)sulfonyl]imide ((CF₃SO₂)₂N⁻, TFSI), or bis(fluorosulfonyl)imide ((FSO₂)N⁻, FSI), more preferably functionalized multi-walled carbon nanotubes of general formula (VI), wherein R is methyl and X⁻ is bromide,
c) 5 wt% of stabilizing agent, preferably sodium carboxymethyl cellulose and
d) 5 wt% of binder, expressed as solids, preferably styrene butadiene rubber copolymer.

The aqueous ink usually comprises from 15 wt% to 25 wt% of solids, more preferably 20 wt%, being water the rest up to 100 wt%. The aqueous ink may content small amounts of co-solvents, which can be present, for example, in the binder.

Aqueous inks for manufacturing the cathode of the invention can be prepared using a horizontal ball mill equipped with 10 mm balls of zirconium oxide. The process for preparing the aqueous ink formulation usually comprises the following steps:
1) Dissolving a stabilizing agent, preferably sodium carboxymethyl cellulose (CMC) in water,
2) Mixing the composite material comprising functionalized carbon material of general formula (VI) and sulfur with CMC solution by planetary ball mill technique,
3) Adding conductive additive and mixing by planetary ball mill technique, and
4) Adding aqueous binder and mixing by planetary ball mill technique.

Usually the particle size of the ink is lower than 5 µm, according to a grindometer VF2112 (TQC). Usually the viscosity measured at 25º C is comprised between 50 and 1000 mPa.s, preferably between 250 and 750 mPa.s, more preferably between 400 and 600 mPa.s, and yet more preferably about 500 mPa.s.

### Cathode

The cathode of the invention comprises the functionalized material of general formula (VI) of the invention.

The cathode of the invention also comprises the composite of functionalized carbon material (VI) of the invention and sulfur.

Preferably, the functionalized material of general formula (VI) is functionalized carbon material selected from the group consisting of carbon black, carbon nanotubes, multi-walled carbon nanotubes, activated carbon, graphite powder, graphite fiber, graphene, carbon fiber, carbon cloth, and vitreous carbon, more preferably selected from carbon black and multi-walled carbon nanotubes, wherein R is selected from linear or branched C₁-C₂₀ alkyl, more preferably from linear or branched C₁-C₁₀ alkyl, yet more preferably from linear or branched C₁-C₅ alkyl, yet more preferably from linear C₁-C₄ alkyl, and yet more preferably is methyl. In a preferred embodiment, X⁻ is bromide. In another preferred embodiment, X⁻ is chloride. In another preferred embodiment X⁻ is (CF₃SO₂)₂N⁻. In another preferred embodiment X⁻ is (FSO₂)N⁻. In a preferred embodiment, R is selected from linear or branched C₁-C₂₀ alkyl, and X⁻ is bromide (Br⁻), chloride (Cl⁻), (CF₃SO₂)₂N⁻, or (FSO₂)N⁻, more preferably bromide. In a more preferred embodiment, R is selected from linear or branched C₁₋C₁₀ alkyl, and X⁻ is bromide (Br⁻), chloride (Cl⁻), (CF₃SO₂)₂N⁻, or (FSO₂)N⁻, more preferably bromide. Ina yet more preferred embodiment, R is selected from linear or branched C₁-C₅ alkyl, and X⁻ is bromide (Br⁻), chloride (Cl⁻), (CF₃SO₂)₂N⁻, or (FSO₂)N⁻, more preferably bromide. In a yet more preferred embodiment, R is selected from linear C₁-C₄ alkyl, and X⁻ is bromide (Br⁻), chloride (Cl⁻), (CF₃SO₂)₂N⁻, or (FSO₂)N⁻, more preferably bromide. In a yet more preferred embodiment, R is methyl, and X⁻ is bromide (Br⁻), chloride (Cl⁻), (CF₃SO₂)₂N⁻, or (FSO₂)N⁻, more preferably bromide.

In a preferred embodiment the functionalized carbon material of general formula (VI) is carbon black, R is methyl, and X⁻ is bromide (Br⁻), chloride (Cl⁻), (CF₃SO₂)₂N⁻, or (FSO₂)N⁻, more preferably bromide.

In another embodiment the functionalized carbon material of general formula (VI) is multi-walled carbon nanotubes, R is methyl, and X⁻ is bromide (Br⁻), chloride (Cl⁻), (CF₃SO₂)₂N⁻, or (FSO₂)N⁻, more preferably bromide.

Also part of the invention is the use of the functionalized carbon material of general formula (VI) for preparing a cathode, preferably for lithium sulfur batteries.

The cathode of the invention is manufactured by printing on aluminium foils with the aqueous ink of the invention comprising the composite material comprising functionalized carbon material of general formula (VI) and sulfur, a conductive additive, stabilizing agent, binder, and water, as disclosed above.

The cathode of the invention can be prepared by bar coating of the ink onto aluminium foils, by a process well known in the state of the art. Then, coated aluminium foils are usually dried at about 25º C and atmospheric pressure during a time period comprised from 8 h to 24 h, preferably from 10 h to 16 h, and more preferably about 12 h, following by a vacuum drying (<50mbar) at room temperature during a period of time comprised from 1 h to 5 h, preferably from 2 h to 4 h, and more preferably about 3 h.

Usually the thickness of the coating obtained with the aqueous ink of the invention is about 10 to 40 µm, and the surface conductivity of the coating is about 600 to 1500 mS.cm⁻¹.

### Lithium sulfur battery

A lithium sulfur battery comprising the cathode of the invention forms also part of the object of the invention.

A lithium sulfur battery comprising the functionalized carbon material of general formula (VI) forms also part of the object of the invention.

A Li-S battery comprises a sulfur-carbon composite as a cathode, a lithium metal anode, a porous separator (normally based on polyethylene or polypropylene) and an organic liquid electrolyte with high concentration of a Lithium salt, for example Li⁺ TFSI⁻, as disclosed in Manthiram et al., Rechargeable Lithium-Sulfur Batteries, Chem. Rev., 2014, 114, 11751-11787.

In the present invention, the Li-S battery comprises the sulfur cathode of the invention, a lithium metal anode and an organic liquid electrolyte.

In Example 14, and Figures 1 and 2, it is shown that a Li-S battery including the functionalized carbon material of general formula (VI) surprisingly provides up to 25% more capacity and lower over voltage in comparison to a Li-S battery, which comprises a non-functionalized carbon material in the cathode.

Moreover, functionalized material shows lower polysulfide shuttle and consume of active material with better cycle stability, comparing with pristine materials or other functionalized materials with different groups, such as benzenesulfonic, with the polar group SO₃H, or aniline derivatives, with the polar group NH₂.

Also part of the invention is the use of the functionalized carbon material of general formula (VI) for preparing a lithium sulfur battery.

Next, several examples of the invention are provided for illustrative purposes that are understood to be non-limiting.

### Examples

### Example 1: Preparation of the imidazolium compound of formula (I). R= Me, and X= Br

### a) Preparation of the nitro derivative of formula (Ila)

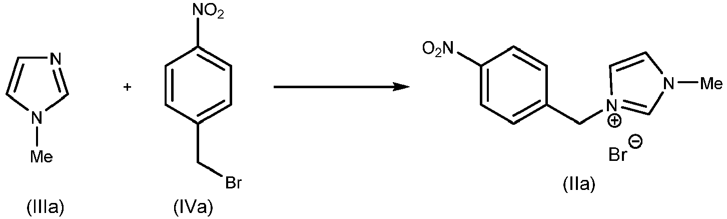

1-Methylimidazole (4.05 mL, 50.4 mmol) was dissolved in dry ethyl acetate (150 mL) in a 3-neck round bottom flask and heated to 65º C under constant stirring and purging with argon. After that, a solution of 4-nitrobenzyl bromide (10 g, 45.8 mmol) in dry ethyl acetate (50 mL) was added dropwise to the heated amine. After the addition, the temperature was maintained at 65° C for 3 hours. The precipitated product was filtrated, washed with cold ethyl acetate (3 x 100 mL) and dried under vacuum at 50° C overnight. Compound of formula (Ila) was obtained as a white solid (12.5 g, 91%)
**UPL**C: 0.83 min;
**IR (ATR)**: 3040, 3009, 1609, 1578, 1559, 1443, 1342 cm⁻¹;
**1H-NMR** (250 MHz, H₂O): δ 8.16 (d, 2H, J = 9 Hz, Hc), 7.46 (d, 2H, J = 9 Hz, Hb), 7.42 (d, 2H, J = 2 Hz, Hb' or Hc'), 7.40 (d, 2H, J = 2 Hz, Hb' or Hc'), 5.46 (s, 2H, CH₂), 3.82 (s, 3H, CH₃); 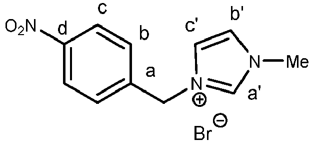 **13C-NMR** (62.5 MHz, H₂O): δ 147.6 [Ca'], 140.7 [Cd], 128.9 [Cb], 124.0 [Cc], 123.8 [Cb' + Cc'], 122.2 [Ca], 51.5 [CH₂], 35.6 [CH₃];
**MS** (FIA): 218; calculated for C₁₁H₁₂N₃O₂⁺: 218;
IC: 25.21 % (w/w) Br⁻; calculated: 26.80 % (w/w) Br⁻.

### b) Reduction of the nitro derivative of formula (Ila)

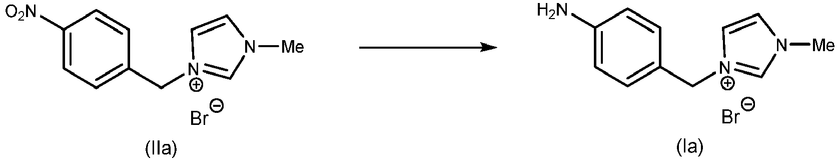

Iron-derived catalyst (800 mg) was activated by adding some drops of water, as disclosed in Lauwiner et al., J. Appl. Catal. A Gen., 1998, 172, 141-148, half an hour before being added to a solution of the nitro derivative of formula (IIa) (6.0 g, 20.1 mmol) in ethanol (200 mL), followed by the addition of hydrazine hydrate (1.49 mL). The reaction mixture was stirred at 60º C during 5 h. Then, the mixture was filtered through celite and the solvent was removed to obtain the desired compound of formula (Ia) without further purification, as a brown solid at room temperature (5.2g, 96%).
UPLC: 0.42 min;
**IR (ATR):** 3302, 3198, 3071, 3030, 3018, 1620, 1608, 1568, 1562,1518 cm-1;
**1H-NMR** (250 MHz, H₂O): δ 7.37 (d, 2H, J = 2 Hz, Hb' or Hc'), 7.35 (d, 2H, J = 2 Hz, Hb' or Hc'), 7.21 (d, 2H, J = 8.5 Hz, Hb), 6.83 (d, 2H, J = 9 Hz, Hc), 5.17 (s, 2H, CH₂), 3.81 (s, 3H, CH₃); 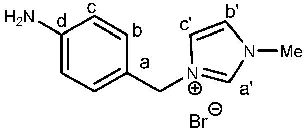 **13C-NMR** (62.5 MHz, H₂O): δ 147.1 [Ca' + Cd], 129.9 [Cb], 123.5 [Cb' + Cc'], 122.3 [Ca], 116.2 [Cc], 52.2 [CH₂], 35.4 [CH₃];
**ESI-MS:** 188; calculated for C₁₁H₁₄N₃⁺: 188;
IC: 26.78 % (w/w) Br⁻; calculated: 29.80 % (w/w) Br⁻.

### Example 2: Preparation of the imidazolium compound of formula (I). R= Me, and X= TFSI

Compound of formula (Ia) was prepared according to the process disclosed in Example 1.

To obtain the compound of the example, compound of formula (Ia) was submitted to a methatesis reaction with Li⁺ TFSI⁻, according to the following process.

To a solution of compound of formula (Ia) (4 g, 14.9 mmol) in water (25 mL) was added a solution of Li⁺ TFSI⁻ (4.33 g, 14.9 mmol) (Sigma-Aldrich) in water (25 mL).

The mixture was stirred 1 h at room temperature. Then, two phases were observed, the crude was extracted with CH₂Cl₂ (200 mL) and the organic layer was washed with water (2 x 20 mL), brine (20 mL), dried over MgSO₄, solvent removed and dried under vacuum at 50ºC overnight, to obtain compound of formula (I), R= Me, and X= TFSI as a brown oil (6.43 g, 92%).
**UPLC:** 0.39 min (imidazolium), 6.33 (TFSI);
**IR (ATR)**: 3458, 3383, 3154, 3115, 3099, 1625, 1612, 1521, 1346 cm⁻¹;
**¹H-NMR** (250 MHz, CD₂Cl₂): δ 8.43 (s, 1H, 7.37 (d, 2H, Ha'), 7.09-7.16 (m, 2H, Hb' + Hc'), 7.06 (d, 2H, *J* = 8.5 Hz, Hb), 6.61 (d, 2H, *J =* 9 Hz, Hc), 5.06 (s, 2H, CH₂), 3.80 (s, 3H, CH₃); 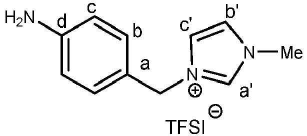 **¹³C-NMR** (62.5 MHz, CD₂Cl₂): δ 148.1 [Cd], 135.1 [Ca'], 130.2 [Cb], 123.4 [Cb'], 121.7 [Cc'], 119.6 [q, *J =* 260 Hz, CF₃], 120.5 [Ca], 115.0 [Cc], 47.0 [CH₂], 36.1 [CH₃];
**ESI-MS:** 188 (calculated for C₁₁H₁₄N₃⁺: 188), 280 (calculated for C₂FₑNO₄S₂: 280);
**IC:** No presence of bromide (lower than 0.1%)

### Examples 3-8: Preparation of functionalized carbon materials of general formula (VI), wherein R = ME, and X = Br

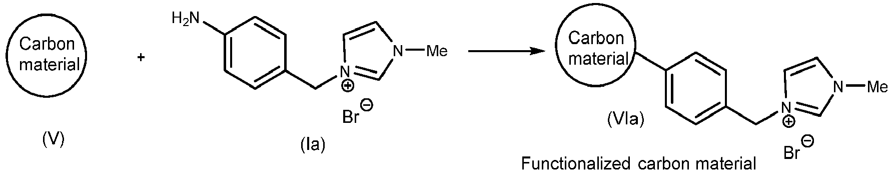

To a carbon material, for example, carbon multi-walled nanotubes (Nanocyl® 7000) or black carbon (Ketjen® Black 600 JD) in a suspension in 0.5 M HCI solution, the imidazolium compound of formula (Ia) was added and the mixture was stirred during 30 minutes at room temperature. After that, sodium nitrite (2 eq.) was added and the mixture was stirred during 24 h. Then, the mixture was filtered and the functionalized carbon was washed with H₂O (100 mL), methanol (100 mL), DMF (100 mL), acetonitrile (100 mL), methanol (100 mL) and H₂O (100 mL). The carbon powder was dried under vacuum at 65º C until no weight changes were observed. The degree of functionalization was calculated by the increment of nitrogen (wt/wt) by elemental analysis.

Different attachment or grafting reactions on two carbon materials (multi-walled carbon nanotubes and black carbon) were carried out modifying the following factors:
1) Carbon material
2) Compound of formula (I)/carbon material ratio
3) Concentration of compound of formula (I)

The functionalization degree obtained under different conditions is shown in Table I:

**TABLE I**

| Example | Carbon material | Compound (I) /carbon ratio (%) | Compound (I) concentration (mM) | Functionalization (%) |
|---|---|---|---|---|
| 3 | Nanocyl® 7000 | 6.0 | 2.24 | 0.8 |
| 4 | Ketjen® Black 600 JD | 6.0 | 2.24 | 1.6 |
| 5 | Nanocyl® 7000 | 6.0 | 7.46 | 1.8 |
| 6 | Ketjen® Black 600 JD | 6.0 | 7.46 | 5.8 |
| 7 | Nanocyl® 7000 | 20.0 | 7.46 | 1.9 |
| 8 | Ketjen® Black 600 JD | 20.0 | 7.46 | 6.6 |

These six examples have been designed according to two 2² factorial designs in order to determine the effects of the different factors.

### a) First 2² factorial design

In this design, the carbon material and the concentration of the compound of general formula (I) are the factors to be checked, whereas the ratio between the compound (I) and the carbon material is maintained constant at 6%. The layout of the design is shown in Table II:

**TABLE II**

| Example | Carbon material | Compound (I) /carbon ratio (%) | Compound (I) concentration (mM) | Functionalization (%) |
|---|---|---|---|---|
| 3 | Nanocyl® 7000 | 6.0 | 2.24 | 0.8 |
| 4 | Ketjen® Black 600 JD | 6.0 | 2.24 | 1.6 |
| 5 | Nanocyl® 7000 | 6.0 | 7.46 | 1.8 |
| 6 | Ketjen® Black 600 JD | 6.0 | 7.46 | 5.8 |

The effects calculated from the results showed that Ketjen® Black 600 JD as carbon material increases the functionalization degree by 2.4 points in comparison to Nanocyl® 7000, and that the increase of the concentration of compound (I) from 2.24 mM to 7.46 mM increases the functionalization degree by 2.6 points. Moreover there is an unexpected synergistic using Ketjen® Black 600 and 7.46 mM.

This increase in the functionalization of carbon black (Ketjen® Black 600) over multi-walled carbon nanotubes (Nanocyl® 7000) can be explained in view of the extremely high surface area of carbon black of about 1400 m²/g, in comparison to the 250-300 m²/g of the multi-walled carbon nanotubes.

### b) Second 2² factorial design

In this design, the carbon material and the ratio between the compound (I) and the carbon material are the factors to be checked, whereas the concentration of the compound of general formula (I) is maintained constant at 7.46 mM. The layout of the design is shown in Table III:

**TABLE III**

| Example | Carbon material | Compound (I) /carbon ratio (%) | Compound (I) concentration (mM) | Functionalization (%) |
|---|---|---|---|---|
| 5 | Nanocyl® 7000 | 6.0 | 7.46 | 1.8 |
| 6 | Ketjen® Black 600 JD | 6.0 | 7.46 | 5.8 |
| 7 | Nanocyl® 7000 | 20.0 | 7.46 | 1.9 |
| 8 | Ketjen® Black 600 JD | 20.0 | 7.46 | 6.6 |

The effects calculated from the results showed that Ketjen® Black 600 JD as carbon material increases the functionalization degree by 4.35 points in comparison to Nanocyl® 7000, and that the ratio between the compound (I) and the carbon material does not have any significant effect on the functionalization degree. In this case, no synergism effect was identified.

### Example 9: Preparation of a cathode for a lithium sulfur battery

### a) Preparation of a composite

Composites were prepared by combining sulfur with the functionalized carbon material of general formula (VI). After thorough homogenization using a mortar, the mixture was transferred into a ceramic crucible and heated to 155° C for 12 h under air to perform the melt infiltration of sulfur. In particular, it was prepared a sulfur composite containing 79 wt% of sulfur and 21 wt% of functionalized carbon material according to Example 6.

### b) Preparation of an aqueous ink

The composition of these inks was as shown in Table IV:

**TABLE IV**

| Component | Function | Solids amount (wt%) |
|---|---|---|
| Sulfur/carbon composite prepared using functionalized carbon material (carbon black) of Example 6 | Active material | 85 |
| Functionalized carbon multi-walled nanotubes of Example 5 | Conductive additive | 5 |
| Sodium carboxymethyl cellulose (MW about 700,000) | Stability agent | 5 |
| Styrene butadiene rubber co-polymer latex (SBR T102) | Binder | 5 |

The amount of solids in the aqueous ink was 20 wt%, being the rest, 80 wt% deionized water.

Cathode aqueous inks were prepared using a horizontal ball mill equipped with 10 mm balls. The process for preparing the aqueous ink formulation comprises the following steps:
1) Dissolving sodium carboxymethyl cellulose (CMC) in water,
2) Mixing functionalized carbon material, according to the invention, with CMC solution by planetary ball mill technique,
3)Adding conductive additive and mixing by planetary ball mill technique, and
4)Adding aqueous binder and mixing by planetary ball mill technique.

The aqueous ink showed a viscosity of about 500 mPa.s measured at 25º C and shear rate of 200 s⁻¹.

### c) Preparation of the cathode

The cathodes were prepared by bar coating of the ink onto aluminium foils. Then, coated aluminium foils were dried at about 25º C and atmospheric pressure during 12 h, following by a vacuum drying (<50mbar) at room temperature during 3 h.

### Example 10: Preparation of a cathode for a lithium sulfur battery

### a) Preparation of a composite

Composites were prepared by combining sulfur with the functionalized carbon material of general formula (VI). After thorough homogenization using a mortar, the mixture was transferred into a ceramic crucible and heated to 155° C for 12 h under air to perform the melt infiltration of sulfur. In particular, it was prepared a sulfur composite containing 79 wt% of sulfur and 21 wt% of functionalized carbon material according to Example 5.

### b) Preparation of an aqueous ink

The composition of these inks was as shown in Table V:

**TABLE V**

| Component | Function | Solids amount (wt%) |
|---|---|---|
| Sulfur/carbon composite prepared using functionalized multi-walled nanotubes of Example 5 | Active material | 85 |
| Functionalized carbon multi-walled nanotubes of Example 5 | Conductive additive | 5 |
| Sodium carboxymethyl cellulose (MW about 700,000) | Stability agent | 5 |
| Styrene butadiene rubber co-polymer latex (SBR T102) | Binder | 5 |

The amount of solids in the aqueous ink was 20 wt%, being the rest, 80 wt% deionized water.

Cathode aqueous inks were prepared according to an analogous process as disclosed in Example 9.

The aqueous ink showed a viscosity of about 500 mPa.s measured at 25º C and shear rate of 200 s⁻¹.

### c) Preparation of the cathode

The cathodes were prepared according to an analogous process as disclosed in Example 9.

### Example 11: Preparation of a comparative lithium sulfur cathode

One comparative cathode was prepared using a composite of sulfur and non-functionalized carbon material (Carbon black) and non-functionalized carbon material (multi-walled carbon nanotubes) as conductive additive, NaCMC and SBR latex according to an analogous procedure as disclosed in Example 9.

### Example 12: Preparation of a comparative lithium sulfur cathode

One comparative cathode was prepared using a composite of sulfur and non-functionalized carbon material (multi-walled carbon nanotubes) and non-functionalized carbon material (multi-walled carbon nanotubes) as conductive additive, NaCMC and SBR latex according to an analogous procedure as disclosed in Example 10.

### Example 13: Test of the cathodes

Cathodes prepared according to the invention (Examples 9 and 10) and comparative cathodes (Examples 11 and 12) were tested according to the following procedure.

Disk-shaped electrodes were punched (15 mm diameter) and CR2016 coin cells were assembled into an argon-filled glove box. The composition of the coin cell was: Lithium 99,9% as anode, polypropylene membrane (Celgard 2500) as separator, stainless steel discs as spacers and 1M LiTFSI - DME:DIOX (1:1) - 0.25M LiNO₃ as electrolyte.

To test the cathodes, galvanostatic cycling was run applying C/5 intensity between 1.8 and 2.6 V vs. Li/Li+ during 10 cycles. The first cycle was performed at 100% DOD (Depth of discharge) and the subsequent at 80% DOD.

It was observed that using imidazolium functionalized materials, an increase of capacity and lower over-voltage. The increase of capacity depends on the carbon material: 25% for carbon black and 80% for multi-walled carbon nanotubes.

In Figure 1 it is shown the performance of a battery prepared from a functionalized carbon material according to Example 9 of the invention, whereas in Figure 2, it is shown the performance of the comparative battery, prepared using non-functionalized carbon materials according to Example 11.

In Figure 3 it is shown the performance of a battery prepared from a functionalized carbon material according to Example 10 of the invention, whereas in Figure 4, it is shown the performance of the comparative battery, prepared using non-functionalized carbon materials according to Example 12.

The performance of the functionalized material according to the invention improves clearly that of the non-functionalized material, because the former show lower polysulfide shuttle and consume of active material with better cycle stability.

## Claims

1. Compound of general formula (I): 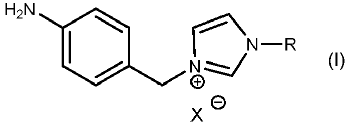 wherein:
- R is linear or branched C₁-C₂₀ alkyl, or alicyclic C₃-C₁₀, and
- X⁻ is bromide (Br⁻), chloride (Cl⁻), bis[(trifluoromethyl)sulfonyl]imide ((CF₃SO₂)₂N⁻, TFSI), or bis(fluorosulfonyl)imide ((FSO₂)N⁻, FSI).

2. Compound according to claim 1, wherein R is selected from linear C₁-C₄ alkyl.

3. Compound according to any of the claims 1 to 2, wherein X⁻ is bromide.

4. A process for preparing compound of general formula (I) according to any of claims 1 to 3, that comprises the reaction of compound of general formula (III) 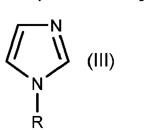 with compound of general formula (IV) 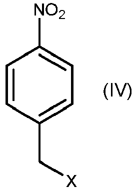 wherein:
- R is linear or branched C₁-C₂₀ alkyl, or alicyclic C₃-C₁₀, and
- X⁻ is bromide (Br⁻), chloride (Cl⁻),
to produce compound of general formula (II) 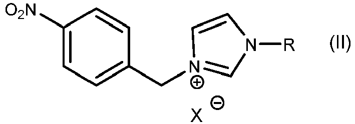 , which is subsequently reduced to produce compound of general formula (I) 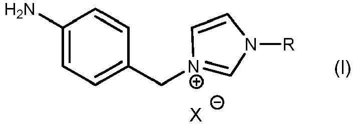 wherein when X⁻ is bis[(trifluoromethyl)sulfonyl]imide ((CF₃SO₂)₂N⁻, TFSI), or bis(fluorosulfonyl)imide ((FSO₂)N⁻, FSI) in compound of general formula (I), the process comprises a further step wherein the halogenide anion, bromide or chloride, is substituted by TFSI or FSI in a methatesis reaction in the presence of an alkaline salt of TFSI or FSI.

5. Process for preparing a functionalized carbon material of general formula (VI) 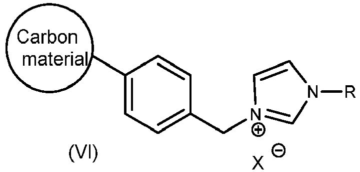 that comprises the reaction of compound of general formula (I) 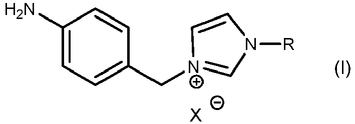 according to any of claims 1 to 3, with a carbon material.

6. Process according to claim 5, wherein the carbon material is selected from the group consisting of carbon black, carbon nanotubes, multi-walled carbon nanotubes, activated carbon, graphite powder, graphite fiber, graphene, carbon fiber, carbon cloth, and vitreous carbon.

7. Functionalized carbon material of general formula (VI) obtainable according to the process of any of claims 5 to 6.

8. Composite comprising the functionalized material of general formula (VI) according to claim 7 and sulfur.

9. Aqueous ink that comprises:
f) the composite according to claim 8,
g) a conductive additive,
h) a stabilizing agent,
i) a binder, and
j) water as a solvent

10. Aqueous ink according to claim 9, wherein the conductive additive is a functionalized carbon material of general formula (VI) according to claim 7.

11. Aqueous ink according to any of claims 9 to 10, wherein the stabilizing agent is selected form the group consisting of sodium carboxymethyl cellulose, xanthan gum, hydroxypropylmethyl cellulose, carbomer, polyvinylpyrrolidone, and mixtures thereof.

12. Cathode comprising the functionalized carbon material of general formula (VI) according to claim 7.

13. Use of the functionalized carbon material of general formula (VI) according to claim 7 for preparing a cathode.

14. Lithium sulfur battery comprising the functionalized carbon material of general formula (VI) according to claim 7.

15. Use of the functionalized carbon material of general formula (VI) according to claim 7 for preparing a lithium sulfur battery.

## Patentansprüche

1. Verbindung der allgemeinen Formel (I): 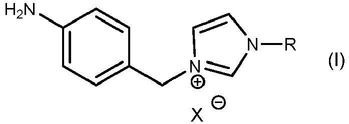 wobei:
- R lineares oder verzweigtes C₁-C₂₀-Alkyl oder alicyclisches C₃-C₁₀ ist, und
- X⁻ Bromid (Br), Chlorid (Cl⁻), Bis[(trifluormethyl)sulfonyl]imid ((CF₃SO₂)₂N⁻, TFSI) oder Bis(fluorsulfonyl)imid ((FSO₂)N⁻, FSI) ist.

2. Verbindung nach Anspruch 1, wobei R ausgewählt ist aus linearem C₁-C₄-Alkyl.

3. Verbindung nach einem der Ansprüche 1 bis 2, wobei X⁻ Bromid ist.

4. Verfahren zum Herstellen einer Verbindung der allgemeinen Formel (I) nach einem der Ansprüche 1 bis 3, umfassend die Reaktion einer Verbindung der allgemeinen Formel (III), 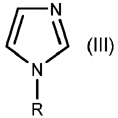 mit einer Verbindung der allgemeinen Formel (IV) 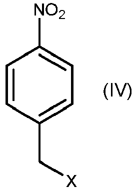 wobei:
- R lineares oder verzweigtes C₁-C₂₀-Alkyl oder alicyclisches C₃-C₁₀ ist, und
- X⁻ Bromid (Br), Chlorid (Cl⁻) ist,
um eine Verbindung der allgemeinen Formel (II) zu erzeugen, 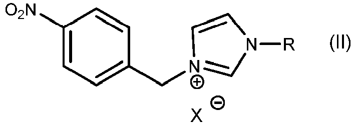 die anschließend reduziert wird, um eine Verbindung der allgemeinen Formel (I) zu erzeugen 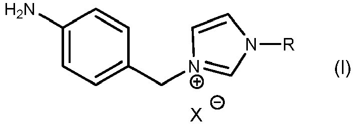 wobei, wenn X⁻ Bis[(trifluormethyl)sulfonyl]imid ((CF₃SO₂)₂N⁻, TFSI) oder Bis(fluorsulfonyl)imid ((FSO₂)N⁻, FSI) in einer Verbindung der allgemeinen Formel (I) ist, das Verfahren einen weiteren Schritt umfasst, wobei das Halogenidanion, Bromid oder Chlorid in einer Methatesis-Reaktion in Gegenwart eines alkalischen Salzes von TFSI oder FSI durch TFSI oder FSI substituiert wird.

5. Verfahren zum Herstellen eines funktionalisierten Kohlenstoffmaterials der allgemeinen Formel (VI), 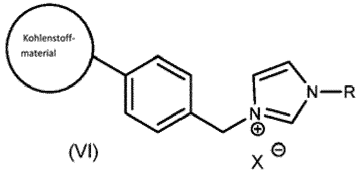 umfassend die Reaktion einer Verbindung der allgemeinen Formel (I) 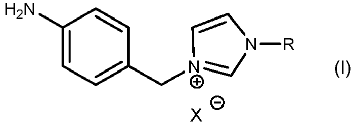 nach einem der Ansprüche 1 bis 3 mit einem Kohlenstoffmaterial.

6. Verfahren nach Anspruch 5, wobei das Kohlenstoffmaterial ausgewählt ist aus der Gruppe bestehend aus Ruß, Kohlenstoffnanoröhren, mehrwandigen Kohlenstoffnanoröhren, Aktivkohle, Graphitpulver, Graphitfaser, Graphen, Kohlenstofffaser, Kohlenstofftuch und Glaskohlenstoff.

7. Funktionalisiertes Kohlenstoffmaterial der allgemeinen Formel (VI), erhältlich gemäß dem Verfahren nach einem der Ansprüche 5 bis 6.

8. Verbundstoff, umfassend das funktionalisierte Material der allgemeinen Formel (VI) nach Anspruch 7 und Schwefel.

9. Wässrige Tinte, umfassend:
f) den Verbundstoff nach Anspruch 8,
g) ein leitfähiges Additiv,
h) ein Stabilisierungsmittel,
i) ein Bindemittel und
j) Wasser als Lösungsmittel.

10. Wässrige Tinte nach Anspruch 9, wobei das leitfähige Additiv ein funktionalisiertes Kohlenstoffmaterial der allgemeinen Formel (VI) nach Anspruch 7 ist.

11. Wässrige Tinte nach einem der Ansprüche 9 bis 10, wobei das Stabilisierungsmittel ausgewählt ist aus der Gruppe bestehend aus Natriumcarboxymethylcellulose, Xanthangummi, Hydroxypropylmethylcellulose, Carbomer, Polyvinylpyrrolidon und Mischungen davon.

12. Kathode, umfassend das funktionalisierte Kohlenstoffmaterial der allgemeinen Formel (VI) nach Anspruch 7.

13. Verwendung des funktionalisierten Kohlenstoffmaterials der allgemeinen Formel (VI) nach Anspruch 7 zum Herstellen einer Kathode.

14. Lithium-Schwefel-Batterie, umfassend das funktionalisierte Kohlenstoffmaterial der allgemeinen Formel (VI) nach Anspruch 7.

15. Verwendung des funktionalisierten Kohlenstoffmaterials der allgemeinen Formel (VI) nach Anspruch 7 zum Herstellen einer Lithium-Schwefel-Batterie.

## Revendications

1. Composé de formule générale (I) : 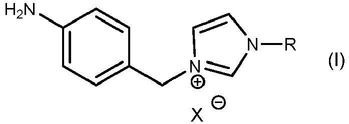 dans lequel :
- R est un alkyle en C₁-C₂₀ linéaire ou ramifié, ou alicyclique en C₃-C₁₀, et
- X⁻ est un bromure (Br⁻), un chlorure (Cl⁻), bis[(trifluorométhyl)sulfonyl]imide ((CF₃SO₂)₂N⁻, TFSI), ou bis(fluorosulfonyl)imide ((FSO₂) N⁻, FSI).

2. Composé selon la revendication 1, dans lequel R est choisi dans l'alkyle en C₁-C₄ linéaire.

3. Composé selon l'une quelconque des revendications 1 à 2, dans lequel X⁻ est un bromure.

4. Procédé de préparation de composé de formule générale (I) selon l'une quelconque des revendications 1 à 3, qui comprend la réaction de composé de formule générale (III) 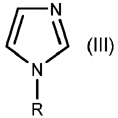 avec un composé de formule générale (IV) 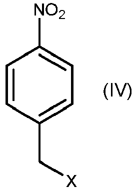 dans lequel :
- R est un alkyle en C₁-C₂₀ linéaire ou ramifié, ou alicyclique en C₃-C₁₀, et
- X⁻ est un bromure (Br⁻), un chlorure (Cl⁻),
pour produire un composé de formule générale (II) 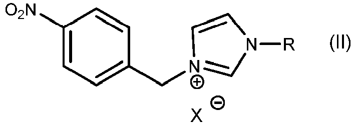 , qui est ensuite réduit pour produire un composé de formule générale (I) 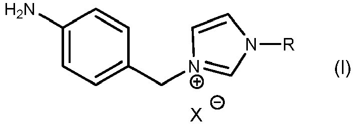 dans lequel quand X⁻ est bis[(trifluorométhyl)sulfonyl]imide ((CF₃SO₂)₂N⁻, TFSI), ou bis(fluorosulfonyl)imide ((FSO₂) N⁻, FSI) dans un composé de formule générale (I), le procédé comprend une étape supplémentaire dans laquelle l'anion halogénure, le bromure ou le chlorure, est substitué par TFSI ou FSI dans une réaction de métathèse en présence d'un sel alcalin de TFSI ou FSI.

5. Procédé de préparation d'une matière carbonée fonctionnalisée de formule générale (VI) 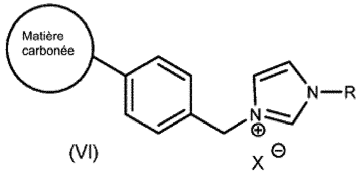 qui comprend la réaction du composé de formule générale (I) 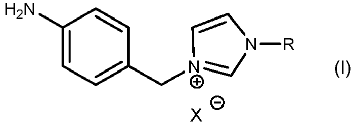 selon l'une quelconque des revendications 1 à 3, avec une matière carbonée.

6. Procédé selon la revendication 5, dans lequel la matière carbonée est choisie dans le groupe constitué de noir de carbone, nanotubes de carbone, nanotubes de carbone à parois multiples, charbon actif, poudre de graphite, fibre de graphite, graphène, fibre de carbone, tissu de carbone, et carbone vitreux.

7. Matière carbonée fonctionnalisée de formule générale (VI) pouvant être obtenue selon le procédé de l'une quelconque des revendications 5 à 6.

8. Composite comprenant la matière fonctionnalisée de formule générale (VI) selon la revendication 7 et du soufre.

9. Encre aqueuse qui comprend :
f) le composite selon la revendication 8,
g) un additif conducteur,
h) un agent stabilisant,
i) un liant, et
j) de l'eau comme solvant

10. Encre aqueuse selon la revendication 9, dans laquelle l'additif conducteur est une matière carbonée fonctionnalisée de formule générale (VI) selon la revendication 7.

11. Encre aqueuse selon l'une quelconque des revendications 9 à 10, dans laquelle l'agent stabilisant est choisi dans le groupe constitué de carboxyméthylcellulose sodique, gomme de xanthane, hydroxypropylméthylcellulose, carbomère, polyvinylpyrrolidone, et des mélanges de ceux-ci.

12. Cathode comprenant la matière carbonée fonctionnalisée de formule générale (VI) selon la revendication 7.

13. Utilisation de la matière carbonée fonctionnalisée de formule générale (VI) selon la revendication 7 pour la préparation d'une cathode.

14. Batterie lithium-soufre comprenant la matière carbonée fonctionnalisée de formule générale (VI) selon la revendication 7.

15. Utilisation de la matière carbonée fonctionnalisée de formule générale (VI) selon la revendication 7 pour la préparation d'une batterie lithium-soufre.
